# EUROPEAN PATENT SPECIFICATION

(11) **EP 3 799 879 B1**
(45) Date of publication and mention of the grant of the patent: **04.06.2025**
(21) Application number: 20176001.4
(22) Date of filing: 08.01.2016
(51) Int. Cl.: A61K 38/19, C12N 15/87, A61K 48/00, A61K 38/20, C07K 14/525, A61P 35/00, A61K 38/17

(54) **GENE THERAPY USING CO-STIMULATORY MOLECULE AND CYTOKINE FOR THE TREATMENT OF MALIGNANCIES**
GENTHERAPIE MIT CO-STIMULIERENDE MOLEKÜLE UND CYTOKINE ZUR BEHANDLUNG VON MALIGNOMEN
THÉRAPIE GÉNIQUE UTILISANT UNE MOLÉCULE CO-STIMULANTE ET UNE CYTOKINE POUR LE TRAITEMENT DE TUMEURS MALIGNES

(30) Priority: 09.01.2015 US 201562101850 P; 27.02.2015 US 201562126300 P
(43) Date of publication of application: 07.04.2021
(62) Divisional of application: 16735524.7
(73) Proprietor: Grand Decade Developments Limited, Central, Hong Kong (HK)
(72) Inventor: PIERCE, Robert H., Seattle WA 98103 (US); WRIGHT, Jocelyn, H., Poulsbo, WA Washington 98370 (US)
(74) Representative: Murgitroyd & Company

(56) References cited:
- WO-A1-2013/090296
- WO-A1-96/39226
- WO-A2-2004/110371
- US-A1- 2011 142 887
- US-A1- 2011 250 230
- US-B1- 8 802 643
- STONE GEOFFREY WILLIAM ET AL: "Treatment of established murine tumors by peritumoral injections of plasmid DNA for soluble, multimeric CD40 ligand and GITR ligand", THE FASEB JOURNAL, FEDERATION OF AMERICAN SOCIETIES FOR EXPERIMENTAL BIOLOGY, US, vol. 19, no. 5, Suppl. S, Part 2, 28 February 2005 (2005-02-28), pages A1403 - A1404, XP009505316, ISSN: 0892-6638
- KNEE DEBORAH A ET AL: "Rationale for anti-GITR cancer immunotherapy", EUROPEAN JOURNAL OF CANCER, vol. 67, November 2016 (2016-11-01), pages 1 - 10, XP002782307

## Description

### FIELD OF THE INVENTION

The present invention provides for the intratumoral delivery of immunomodulators. In particular, it provides delivery of co-stimulatory molecules by intratumoral electroporation.

### BACKGROUND OF THE INVENTION

Immunotherapy has recently drawn attention as a fourth method following surgery, chemotherapy and radiation therapy for treating tumors. Since immunotherapy utilizes the immunity inherent to humans, it is said that the physical burden on patients are less in immunotherapy than those in other therapies. The therapeutic approaches known as immunotherapies include: cell transfer therapy in which cells such as lymphokine-activated cells, natural killer T-cells or γδT cells obtained, for example, from exogenously-induced cytotoxic T-lymphocytes (CTLs) or peripheral blood lymphocytes by expansion culture using various method are transferred; dendritic cell-transfer therapy or peptide vaccine therapy by which in vivo induction of antigen-specific CTLs is expected; Th1 cell therapy; and immune gene therapy in which genes expected to have various effects are introduced ex vivo into the above-mentioned cells to transfer them in vivo. In these immunotherapies, CD4-positive T cells and CD8-positive T cells are traditionally known to play a critical role.

CD8-positive T cells are major effector cells that are capable of directly destroying tumor cells in vivo and in vitro. These cells are strictly specific to antigen peptides presented by MHC Class 1 molecules. In contrast, antigen specificities of NKT cells are not so strict, and they are considered to be effector cells that show intrinsic immune responses.

CD4-positive T cells are considered to have a fundamental role to regulate anti-tumor immune responses through a plurality of mechanisms although they do not destroy tumors directly. CD4-positive T cells that have recognized a tumor-antigen peptide represented by MHC Class II molecules promote the activation and proliferation of CTL through the interaction with antigen-presenting cells (APCs).

In contrast, CD25-positive/CD4-positive cells (regulatory T cells: Treg) have been shown to inhibit the anti-tumor immune responses and progression of various autoimmune diseases (see US 2003049696 and S. Sakaguchi et al., Immunol. Rev. 182 (2001), pp 18-32). Specifically, since regulatory T cells suppress the activity of cytotoxic CD8-positive T cells through the control of the helper function by targeting CD4-positive T cells, some tumors are considered to utilize this system for their proliferation, thereby avoiding attack of the immune system.

GITR, which has been found as a gene expressed in regulatory T cells ("Tregs"; see S. Sakaguchi et al., Immunol. Rev. 182 (2001), pp 18-32), is a cell surface transmembrane protein receptor and a member of the tumor necrosis factor receptor (TNFR) superfamily. GITR has been shown to be constitutively present on Tregs and activated T cells. GITR binds to another transmembrane protein referred to as GITR ligand (hereinafter referred to as "GITR-L"). Agonistic antibodies against GITR have been shown to abrogate the immunosuppressant activity of regulatory T cells, suggesting that GITRL plays a functional role in regulating the activity of regulatory T cells via GITR (see McHugh et al., Immunity 16 (2002), PP 311-23). Similarly, other co-stimulatory molecules such as CD137, CD134, CD40L, CD27, etc. also function to stimulate immunity.

In vivo electroporation is a gene delivery technique that has been used successfully for efficient delivery of plasmid DNA to many different tissues. Studies have reported the administration of in vivo electroporation for delivery of plasmid DNA to B16 melanomas and other tumor tissues. Systemic and local expression of a gene or cDNA encoded by a plasmid can be obtained with administration of in vivo electroporation. Use of in vivo electroporation enhances plasmid DNA uptake in tumor tissue, resulting in expression within the tumor, and delivers plasmids to muscle tissue, resulting in systemic expression of secreted proteins, such as cytokines (see, e.g., US8026223).

It has been shown that electroporation can be used to transfect cells in vivo with plasmid DNA. Recent studies have shown that electroporation is capable of enhancing delivery of plasmid DNA as an antitumor agent. Electroporation has been administered for treatment of hepatocellular carcinomas, adenocarcinoma, breast tumors, squamous cell carcinoma and B16.F10 melanoma in rodent models. The B16.F10, CT-26, and MC-38 murine syngeneic tumor models has been used extensively for testing potential immunotherapy protocols for the delivery of an immudulatory molecule including cytokines either as recombinant protein or by gene therapy.

Various protocols known in the art can be utilized for the delivery of plasmid encoding a co-stimulatory agonist utilizing in vivo electroporation for the treatment of cancer. The protocols known in the art describe in vivo electroporation mediated cytokine based gene therapy, both intratumor and intramuscular, utilizing low-voltage and long-pulse currents.

Accordingly, what is needed in the art is an electroporation protocol for the delivery of a plasmid encoding a co-stimulatory agonist, such as a GITRL, and CD137 and CD134 agonist, that will provide substantially improved results in the regression of cancer tumors, such as melanoma, while also substantially improving the long-term survival rates.

### BRIEF DESCRIPTION OF THE DRAWINGS

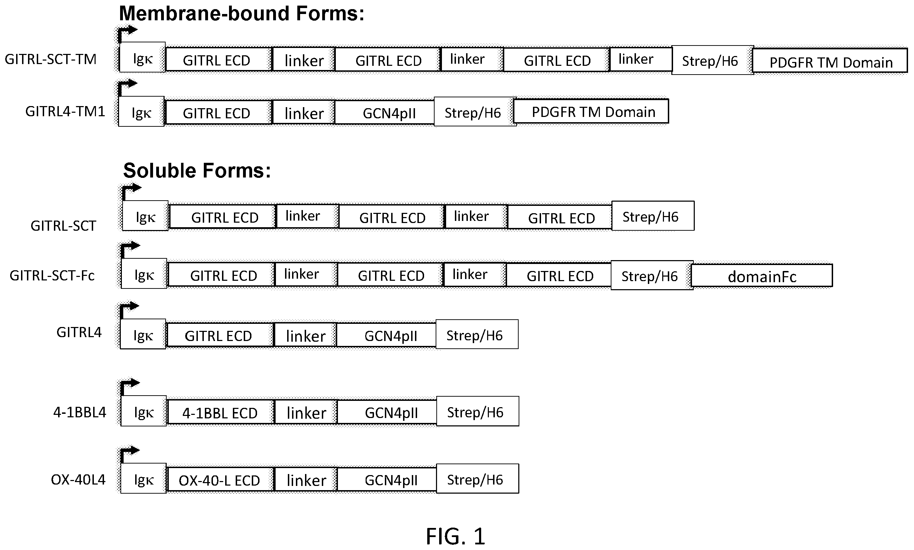

Figure 1 shows the structure of recombinant membrane-bound and soluble human GITRL, OX40L, and 4-1BBL constructs. The recombinant genes shown are inserted in the pUMVC3 plasmid backbone. The soluble proteins containing a single extracellular domains (ECD) and a GCN4pll trimerization motif are denoted: GITRL4, OX40L4, and 4-1BBL4. The soluble proteins with three consecutive ECDs are denoted Single Chain Trimer (SCT) with and without one of three Fc antibody domains added (human IgG1, Mouse igG1, and mouse IgG2a). Membrane bound forms containing a heterologous transmembrane domain (TM) are denoted GITRL4-TM1 and SCT-TM. Identical forms were also created with the mouse ECD in place of the human ECD for pre-clinical studies.

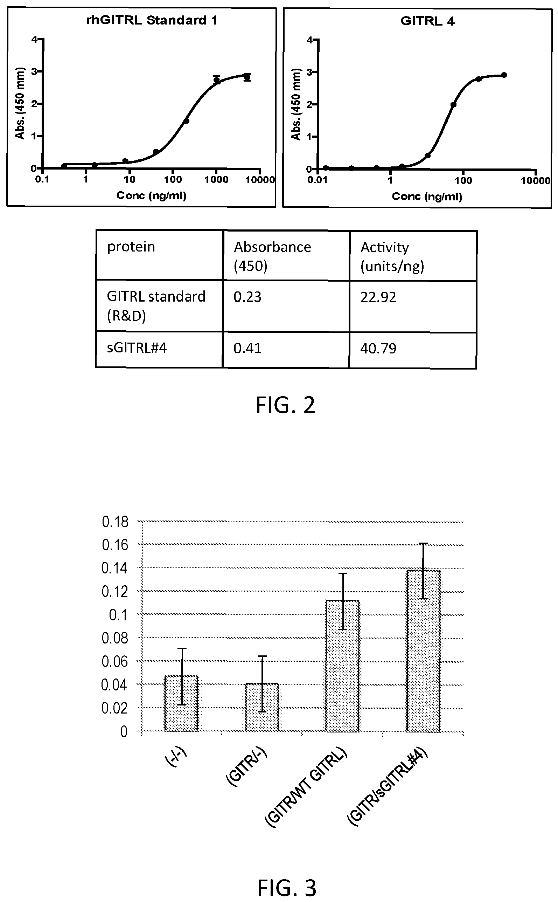

Figure 2 shows a functional ELISAs showing binding of soluble recombinant GITRL4 proteins to soluble GITR-Fc fusion protein.
Figure 3 shows the recombinant GITR ligand-dependent signaling from GITR to NFkB-driven luciferase gene expression in a HEK 293 co-transfection assay. The bar graph shows the relative luciferase activity units in cell lysates. The values are the mean +/- SD for the activity measured from three separate co-transfections lysates.

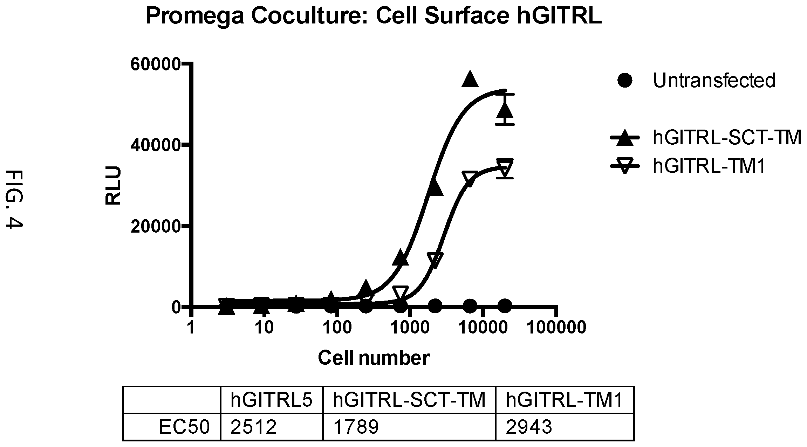

Figure 4 shows the activity of cell-surface recombinant GITRL proteins in co-culture with Jurkat reporter cells from a Promega GITR potency assay. RLU is the relative luciferase units. EC50s are expressed as the number of GITRL-transduced cells in the co-culture assay

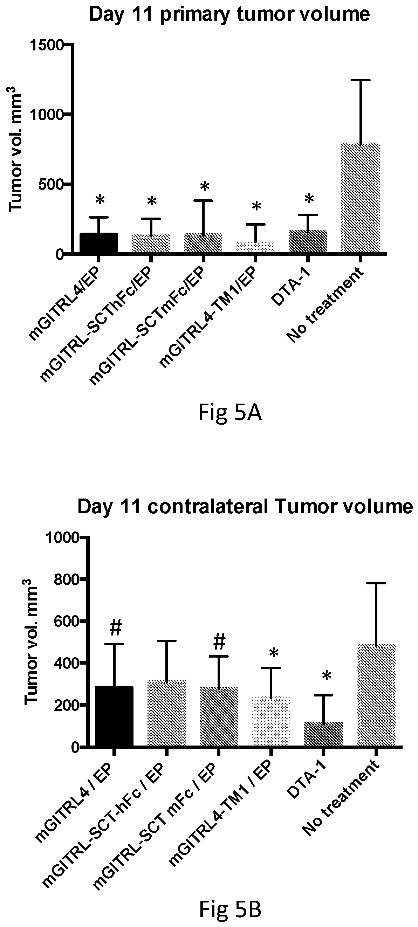

Figure 5 shows the reduction in primary, treated (Figure 5A) and contralateral, untreated (Figure 5B) CT26 subcutaneous tumors in BALB/c mice with intratumoral electroporation of plasmids encoding recombinant mouse GITRL proteins. Untreated tumors and tumors from mice treated systemically with GITR agonist antibody, DTA-1 is shown for comparision. Tumor volume measurements are graphed as means with SD; * = p < 0.01 *vs.* No treatment; # = p < 0.05 *vs.* No Treatment.

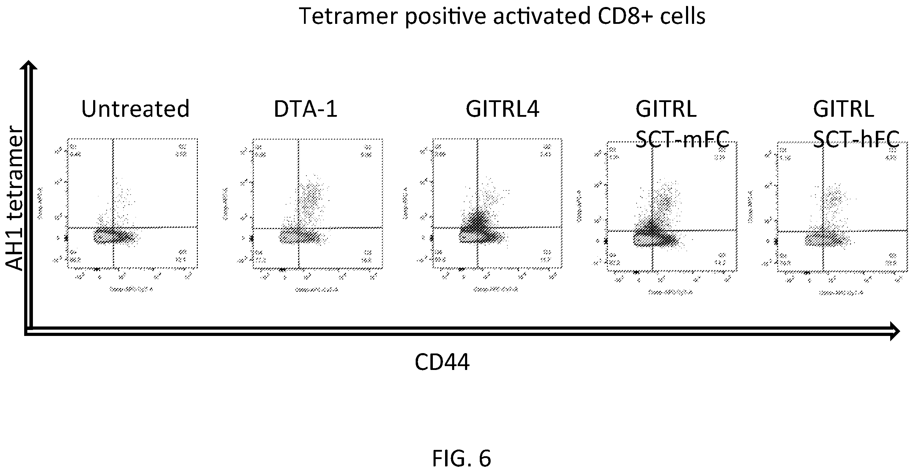

Figure 6 shows the intratumoral electroporation of plasmids encoding mouse GITRL proteins increased AH1-dextramer binding CD8+CD44+ T cells in the spleen 4 days after a single treatment. Shown are 2-dimentional dot plots with log fluorescence intensity staining with anti-CD44 (x-axis) and AHI peptide bound to detramers (y-axis). Splenocytes from untreated and DTA-1 treated mice are shown for comparison. Data presented in each graph are of splenocytes from a single mouse.

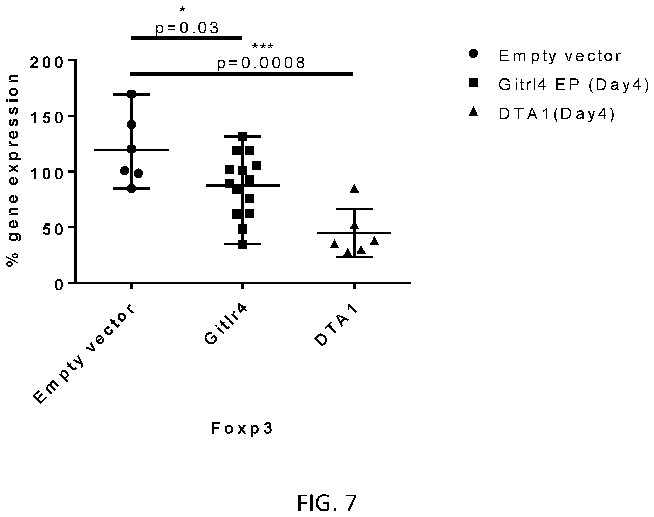

Figure 7 shows the intratumoral electroporation of plasmids encoding mouse GITRL proteins decreased Foxp3 gene expression in tumor infiltrating lymphocytes as compared to electroporation with pUMVC3 empty vector control. Each dot represented triplicate RT-PCR values from a single mouse. Total RNA was isolated from multiple mice with CT26 tumors: n=6 for EP/empty vector and DTA-1 cohorts, and n=14 for EP/GITRL4 cohort. % Gene expression is relative to the average value for empty vector control cohort.

### SUMMARY OF THE INVENTION

The present invention provides a plasmid for use in a method for the treatment of malignancies, wherein the administration of the plasmid encoding for a therapeutic co-stimulatory protein and a nucleic acid encoding at least one immunostimulatory cytokine, in combination with electroporation has a therapeutic effect on primary tumors as well as distant tumors and metastases.

The present invention provides a plasmid for use in a method of treating a subject having a cancerous tumor, the method comprising: injecting the cancerous tumor with an effective dose of the plasmid coding for a therapeutic protein and a nucleic acid encoding at least one immunostimulatory cytokine; and administering electroporation therapy to the tumor, the electroporation therapy further comprising the administration of at least one voltage pulse of 200 V/cm to 1500 V/cm over a pulse width of 100 microseconds to 20 milliseconds. In certain embodiments, the cancerous tumor is melanoma. The plasmid coding for the therapeutic protein is a plasmid coding for a membrane bound form of a co-stimulatory molecule, and the co-stimulatory molecule is selected from GITR, CD137, and CD134 agonists, wherein the agonist is a membrane bound form of GITR-L, CD137-L or CD134-L. In a further embodiment, the plasmid further encodes the at least one immunostimulatory cytokine, which can be selected from IL-12, IL-15, and a combination of IL-12 and IL-15. The voltage pulse delivered to the tumor is from 200V/cm to 1500V/cm.

The present invention provides the plasmid for use in a method of treating a subject having a cancerous tumor, the method comprising: administering a first treatment at time T1, wherein the first treatment further comprises injecting the cancerous tumor with a first effective dose of the plasmid coding for the therapeutic protein and administering a first electroporation therapy to the tumor at time T1, the first electroporation therapy further comprising the administration of at least one voltage pulse having a duration of 100 microseconds to 20 milliseconds; and administering a second treatment at time T2, wherein time T2 is a time later than time T1, wherein the second treatment further comprises injecting the cancerous tumor with a second effective dose of the plasmid coding for the therapeutic protein and administering a second electroporation therapy to the tumor at time T2, the second electroporation therapy further comprising the administration of at least one voltage pulse having a duration of 100 microseconds to 20 milliseconds. In certain embodiments, the cancerous tumor is melanoma. The therapeutic protein is a co-stimulatory molecule selected from a membrane bound form of GITR, CD137 and CD134 agonists, wherein the agonist is a membrane bound form of GITR-L, CD137-L or CD134-L. In another embodiment, the plasmid further encodes the at least one immunostimulatory cytokine, chosen from IL-12, IL-15, and a combination of IL-12 and IL-15. The voltage pulse delivered to the tumor is from 200V/cm to 1500V/cm. In further embodiment, a third treatment is added at time T3, wherein time T3 is a time later than time T2, wherein the third treatment further comprises injecting the cancerous tumor with a third effective dose of the plasmid coding for the therapeutic protein and administering a third electroporation therapy to the tumor, the third electroporation therapy further comprising the administration of at least one voltage pulse having a duration of 100 microseconds to 20 milliseconds. The invention involves injecting an effective dose of the plasmid encoding for the therapeutic protein into the cancerous tumor of the subject; and administering electroporation to the subject intratumorally using at least one low voltage pulse having a pulse width of 100 microseconds to 20 milliseconds and the voltage pulse delivered to the tumor is from 200V/cm to 1500V/cm.

The present invention provides the plasmid for use in a method of treating a subject having a cancerous tumor, the method comprising: administering a first treatment at time T1, wherein the first treatment further comprises injecting the cancerous tumor with a first effective dose of the plasmid coding for the therapeutic protein and administering a first electroporation therapy to the tumor at time T1, the first electroporation therapy further comprising the administration of at least one voltage pulse having a duration of 100 microseconds to 20 milliseconds; administering a second treatment at time T2, wherein time T2 is a time later than time T1, wherein the second treatment further comprises injecting the cancerous tumor with a second effective dose of the plasmid coding for the therapeutic protein and administering a second electroporation therapy to the tumor at time T2, the second electroporation therapy further comprising the administration of at least one voltage pulse having a duration of 100 microseconds to 20 milliseconds; and administering a third treatment at time T3, wherein time T3 is a time later than time T2, wherein the third treatment further comprises injecting the cancerous tumor with a third effective dose of the plasmid coding for the therapeutic protein and administering a third electroporation therapy to the tumor, the third electroporation therapy further comprising the administration of at least one high voltage pulse having a duration of 100 microseconds to 20 milliseconds. In certain embodiments, the cancerous tumor is melanoma. The therapeutic protein is a co-stimulatory molecule, selected from a membrane bound form of GITR, CD137 and CD134 agonists, wherein the agonist is a membrane bound form of GITR-L, CD137-L or CD134-L. In a further embodiment, the plasmid encodes at least one immunostimulatory cytokine, selected from IL-12, IL-15, and a combination of IL-12 and IL-15. The voltage pulse delivered to the tumor is from 200V/cm to 1500V/cm.

### DETAILED DESCRIPTION OF THE INVENTION

As used herein, including the appended claims, the singular forms of words such as "a," "an," and "the," include their corresponding plural references unless the context clearly dictates otherwise.

### Definitions

As used herein, "co-stimulatory molecule" refers to a group of immune cell surface receptor/ligands which engage between T cells and antigen presenting cells and generate a stimulatory signal in T cells which combines with the stimulatory signal (i.e., "co-stimulation") in T cells that results from T cell receptor ("TCR") recognition of antigen on antigen presenting cells. Co-stimulatory molecules include, but are not limited to, agonists of GITR, CD137, CD134, CD40L, CD27, and the like. The plasmid for use in the present invention codes for a membrane bound form of an agonist of GITR, CD137 or CD134, wherein the agonist is a membrane form of GITR-L, CD137-L or CD134-L.

As used herein, "co-stimulator of T cells activation" refers to the ability of a co-stimulatory ligand to bind and to activate T cells that have been activated via TCR. Co-stimulatory activation can be measured for T cells by the production of cytokines as is well known and by proliferation assays such as are well known and described in the examples. The soluble form of a co-stimulatory molecule which is biologically active also may be tested for binding to the cognate receptor on activated T cells.

As used herein, a soluble form of a co-stimulatory molecule "derived from an antigen presenting cell" refers to a co-stimulatory molecule normally expressed by B cells, macrophages, monocytes, dendritic cells and other such antigen presenting cells, which has been engineered as described herein to render it soluble. Preferred soluble co-stimulatory molecules derived from an antigen presenting cell include any of GITR-L, CD137-L, CD134-L (a.k.a. OX40-L), CD40, CD28. The soluble form of a co-stimulatory molecule derived from an antigen presenting cell retains the ability of the native co-stimulatory molecule to bind to its cognate receptor/ligand on T cells and stimulate T cell activation.

The term "co-stimulatory molecule agonist" includes soluble co-stimulatory molecules and agonists of co-stimulatory molecule binding partners. For example, the binding partner of GITR-L is GITR. Agonists of GITR can include agonistic GITR antibodies, GITR-L polypeptides, including multimeric soluble and transmembrane forms, GITRL mimetics, or other molecules that engage and induce biological activity of GITR.

As used herein, the term glucocorticoid-induced tumor necrosis factor receptor "GITR" ligand (a.k.a. GITR-L, TNFSF18 (tumor necrosis factor (ligand) superfamily, member 18)) refers to a specific molecule associated with this name and any other molecules that have biological function as co-stimulatory molecules that share at least 80% amino acid sequence identity, preferably at least 90% sequence identity, more preferably at least 95% sequence identity and even more preferably at least 98% sequence identity with GITR-L as defined in Swiss Prot Id. no. Q9UNG2).

As used herein, the term "CD137-L" or "agonist of CD137" (a.k.a. 4-1 BB ligand or TNFL9) refers to a specific molecule associated with this name and any other molecules that have biological function as co-stimulatory molecules that share at least 80% amino acid sequence identity, preferably at least 90% sequence identity, more preferably at least 95% sequence identity and even more preferably at least 98% sequence identity with human CD137-L as defined in Swiss Prot Id. no. P41273.

Human CD137-L is a type II membrane protein that contains 254 amino acids (no signal sequence) (see sequence in Swiss Prot Id no. P41273). The protein contains a cytoplasmic domain at residues 1-28, a transmembrane domain at resides 29-49 and an extracellular domain at residues 50-254. The nucleotide sequence of CD137-L (1645 bp) is available in public databases (see Genbank accession no. NM 003811). CD137-L is described by Alderson et al. (1994) Eur J. Immunol. 24(9):2219-27. CD137-L is expressed on antigen presenting cells including B cells, monocytes, and splenic dendritic cells and T lymphocytes. CD137-L interacts with CD137 on activated T cells.

As used herein, the term "CD134-L" or "agonist of CD134" (a.k.a. OX40 ligand or TNRSF4) refers to a specific molecule associated with this name and any other molecules that have biological function as co-stimulatory molecules that share at least 80% amino acid sequence identity, preferably at least 90% sequence identity, more preferably at least 95% sequence identity and even more preferably at least 98% sequence identity with CD134-L, as defined in Swiss Prot Id. no. P23510.

Human CD134-L is a type II membrane protein that contains 183 amino acids (no signal sequence). The protein contains a cytoplasmic domain at residues 1-23, a transmembrane domain at residues 24-50 and an extracellular domain at residues 51-183. The nucleotide sequence of CD134-L (3510 bp, with the coding sequence being 157-708) is available in public databases (see. Genbank accession no. NM_003326.2). CD134-L is described by Godfry et al., J Exp Med. Aug. 1, 1994; 180(2):757-62. CD134-L is expressed by dendritic cells and other APC and binds to CD134, which is present on activated T cells.

As used herein, the term "CD40" (a.k.a. TNFRSF5 or CD40 ligand receptor) refers to a specific molecule associated with this name and any other molecules that have biological function as co-stimulatory molecules that share at least 80% amino acid sequence identity, preferably at least 90% sequence identity, more preferably at least 95% sequence identity and even more preferably at least 98% sequence identity with CD40 as defined in Swiss Prot Id. no. P25942).

The sequence of human CD40 contains 277 amino acids of which 20 amino acids at the N terminus represent the signal sequence (see sequence in Swiss Prot Id no. P25942). A transmembrane domain is located at resides 194-215 and the cytoplasmic domain is located at residues 216-277. The nucleotide sequence of CD40 (1177 bp) is available in public databases (see Genbank accession no. NM_001250). CD40 and various isoforms are described by Tone et al. Proc. Natl. Acad. Sci. U.S.A. 98 (4), 1751-1756 (2001). CD40 is expressed by monocytes and B cells binds to CD40-L (a.k.a. CD40 ligand or CD153) expressed by activated T cells.

As used herein, "CD28" is a type I transmembrane glycoprotein and is a member of the Immunoglobulin family by virtue of its single Ig variable-like extracellular domain which has a MYPPPY (SEQ ID NO: 639) motif required for binding CD80 and CD86 (Peach et al. (1994) J. Exp. Med. 180: 2049-2058). CD28 has a cysteine residue located after the Ig variable-like domain, which is involved in its homodimerization. The protein sequence of CD28 and a nucleic acid encoding a human CD28 are disclosed, for example, in Harper et al. J. Immunol. (1991) 147: 1037-44. The sequence of a human mRNA encoding CD28 also is disclosed in NCBI Accession No. NM_006139, last updated Apr. 19, 2009, for example. The complete protein sequence of a human CD28 also is disclosed in NCBI Accession No. NP_006130.

The term "cancer" includes a myriad of diseases generally characterized by inappropriate cellular proliferation, abnormal or excessive cellular proliferation. Examples of cancer include but are not limited to, breast cancer, colon cancer, prostate cancer, pancreatic cancer, melanoma, lung cancer, ovarian cancer, kidney cancer, brain cancer, or sarcomas. Such cancers may be caused by, chromosomal abnormalities, degenerative growth and developmental disorders, mitogenic agents, ultraviolet radiation (UV), viral infections, inappropriate tissue expression of a gene, alterations in expression of a gene, or carcinogenic agents.

The term "treatment" includes, but is not limited to, inhibition or reduction of proliferation of cancer cells, destruction of cancer cells, prevention of proliferation of cancer cells or prevention of initiation of malignant cells or arrest or reversal of the progression of transformed premalignant cells to malignant disease or amelioration of the disease.

The term "subject" refers to any animal, preferably a mammal such as a human. Veterinary uses are also intended to be encompassed by this invention.

The terms "electroporation", "electro-permeabilization," or "electro-kinetic enhancement" ("EP") as used interchangeably herein refer to the use of a transmembrane electric field pulse to induce microscopic pathways (pores) in a bio-membrane; their presence allows biomolecules such as plasmids, oligonucleotides, siRNA, drugs, ions, and water to pass from one side of the cellular membrane to the other.

The term "biomolecule" as used herein, encompasses plasmid encoded antibodies, antibody fragments, full length immunomodulatory proteins, soluble domains of membrane anchored molecules, fusion proteins, and the like.

### Antibodies

The present invention provides an immunotherapeutic approach for reducing the size of a tumor or inhibiting the growth of cancer cells in an individual, or reducing or inhibiting the development of metastatic cancer in an individual suffering from cancer. Therapy is achieved by intratumoral delivery of plasmids encoding membrane bound forms of agonists of GITR, CD137 and CD134 using electroporation.

Co-stimulatory agonists may be in the form of antibodies or antibody fragments, both of which can be encoded in a plasmid and delivered to the tumor by electroporation.

The term "antibody" as used herein includes immunoglobulins, which are the product of B cells and variants thereof as well as the T cell receptor (TcR), which is the product of T cells, and variants thereof. An immunoglobulin is a protein comprising one or more polypeptides substantially encoded by the immunoglobulin kappa and lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Also subclasses of the heavy chain are known. For example, IgG heavy chains in humans can be any of IgG1, IgG2, IgG3 and IgG4 subclass.

A typical immunoglobulin structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (VL) and variable heavy chain (VH) refer to these light and heavy chains, respectively.

Antibodies exist as full-length intact antibodies or as a number of well-characterized fragments produced by digestion with various peptidases or chemicals. Thus, for example, pepsin digests an antibody below the disulfide linkages in the hinge region to produce F(ab')2, a dimer of Fab which itself is a light chain joined to VH-CH₁ by a disulfide bond. The F(ab')2 may be reduced under mild conditions to break the disulfide linkage in the hinge region thereby converting the F(ab')2 dimer into an Fab' monomer. The Fab' monomer is essentially a Fab fragment with the hinge region (see, Fundamental Immunology, W. E. Paul, ed., Raven Press, N.Y. (1993), for a more detailed description of other antibody fragments). A Fab fragment and Fc fragment are generated by digesting IgG with papain. Papain cleaves in the hinge region just above the residues involved in interchain S-S bonding, resulting in monovalent Fab fragments and the Fc fragment, which includes two constant region fragments, each containing the lower part of the hinge, CH2 and CH3 domains. The constant region fragments of the Fc are stabilized as a dimer though interchain S-S bonding of the lower residues of the hinge region.

Immunoglobulin "Fc" classically refers to the portion of the constant region generated by digestion with papain. Includes the lower hinge which has the interchain S-S bonds. The term "Fc" as used herein refers to a dimeric protein comprising a pair of immunoglobulin constant region polypeptides, each containing the lower part of the hinge, CH2 and CH3 domain. Such "Fc" fragment may or may not contain S-S interchain bridging in the hinge region. It should be understood that an Fc may be from any Ig class and, as such, may include a CH4 domain such as in the case of IgM. Mutant sequences of an Fc are known such as described by Wines et al., J. Immunol. 2000 May 15; 164(10):5313-8 and may be used herein.

While various antibody fragments are defined in terms of the digestion of an intact antibody, one of skill will appreciate that any of a variety of antibody fragments may be synthesized de novo either chemically or by utilizing recombinant DNA methodology. Thus, the term antibody, as used herein also includes antibody fragments either produced by the modification of whole antibodies or synthesized de novo or antibodies and fragments obtained by using recombinant DNA methodologies.

Recombinant antibodies may be conventional full length antibodies, antibody fragments known from proteolytic digestion, unique antibody fragments such as Fv or single chain Fv (scFv), domain deleted antibodies, and the like. Fragments may include domains or polypeptides with as little as one or a few amino acid deleted or mutated while more extensive deletion is possible such as deletion of one or more domains.

An Fv antibody is about 50 Kd in size and comprises the variable regions of the light and heavy chain. A single chain Fv ("scFv") polypeptide is a covalently linked VH::VL heterodimer which may be expressed from a nucleic acid including VH- and VL-encoding sequences either joined directly or joined by a peptide-encoding linker. See e.g., Huston, et al. (1988) Proc. Nat. Acad. Sci. USA, 85:5879-5883. A number of structures for converting the naturally aggregated, but chemically separated light and heavy polypeptide chains from an antibody V region into an scFv molecule which will fold into a three dimensional structure substantially similar to the structure of an antigen-binding site.

An alternative to the traditional antibody fragments above has been found in a set of unique antibodies produced by the immune systems of camels, llamas, and sharks. Unlike other antibodies, these affinity reagents are composed of only two heavy chains; better yet, a single domain forms the antigen-binding sites for these heavy-chain antibodies. The domains can even be genetically engineered to produce extremely small, very stable single-domain recombinant antibody fragments, called "nanobodies." Plasmids encoding heavy chain only (VHH), single domain antibodies, and nanobodies are also contemplated for intratumoral delivery by electroporation.

### Soluble Agonists

The present invention relates to a plasmid coding for a membrane bound form of GITR, CD137 and CD134 agonists, wherein the agonist is a membrane bound form of GITR-L, CD137-L or CD134-L. As such, the below discussion relating to soluble agonists does not form part of the present invention.

Agonists of co-stimulatory molecules may be soluble molecules such as soluble GITR-L, which comprises at least the extracellular domain (ECD) of GITR-L. Other co-stimulatory molecules will similarly lack transmembrane and intracellular domains, but are capable of binding to their binding partners and eliciting a biological effect. For intratumoral delivery by electroporation, the ECD's will be encoded in an expression vector and will be expressed when delivered to the tumor.

The soluble encoded form of the co-stimulatory molecule may be linked in the expression vector to DNA encoding another protein or polypeptide. Such other polypeptide may be the Fc portion of an immunoglobulin, albumin, or any other type of serum protein or fragment thereof which maintains the solubility of the co-stimulatory molecule. The soluble form of the co-stimulatory molecule may be linked to an immunoglobulin via the heavy and/or light chain, which may be a fragment or a full length heavy or light chain. The immunoglobulin may be an antibody that can target an antigen associated with a cancer cell or tumor. The co-stimulatory molecule may also be expressed on the cell surface of cells, may have the addition of helerologous trimerization domains (e.g. GCN4) or may have point mutations in receptor binding domain that render the ligand more potent (see, e.g., Chattopadhyay et al. (2007) Proc. Natl. Acad. Sci.,104:19452-19457)*.*

The co-stimulatory molecule agonist is delivered as a nucleic acid. Nucleic acid refers to a polynucleotide compound, which includes oligonucleotides, comprising nucleosides or nucleoside analogs that have nitrogenous heterocyclic bases or base analogs, covalently linked by standard phosphodiester bonds or other linkages. Nucleic acids can include RNA, DNA, chimeric DNA-RNA polymers, or analogs thereof. The DNA can be a plasmid expressing a particular co-stimulatory molecule agonist of interest.

The DNA plasmid used for electroporation of encoded co-stimulatory molecules, is one that includes an encoding sequence of a recombinant antigen that is capable of being expressed in a mammalian cell, upon said DNA plasmid entering after electroporation. Preferably, the encoding sequence is a consensus co-stimulatory molecule agonist that elicits an immune response in the target tumor. In some embodiments, the encoding sequence constructs are optimized for mammalian expression, which can include one or more of the following: including the addition of a Kozak sequence, codon optimization, and RNA optimization. These optimized encoding sequences can be subcloned into various commercially available vectors.

### Combination Therapies

It is contemplated that intratumoral electroporation of DNA encoding co-stimulatory agonists can be administered with other therapeutic entities. Table 1 provides possible combinations. Administration of the combination therapies can be achieved by electroporation alone or a combination of electroporation and systemic delivery.

**Table 1: Combination therapies with co-stimulatory agonists**

| **Combination** | **Proposed delivery methods** | | **Method in reference** | **References** |
|---|---|---|---|---|
| *GITRL + CTLA4 agonist antibody ("Ab") or ligand | | 1. Electroporation ('EP") of plasmids encoding ligands for both receptors | Transfection of CTLA-4 agonist Ab gene+GITRL gene into dendritic cells | Pruitt et al., (2011) Eur. J. Immunol. 41: 3553-3563 |
| | | 2. EP of GITRL gene + systemic anti-CTLA-4 | | |
| | | 3. EP of genes encoding anti-CTLA-4 + GITRL | | |
| GITRL + cytokines (i.e. IL-12 or IL-2) | | 1. EP of plasmids encoding both genes | System ic exposure to anti-OX40 agonist Ab + IL-2 cytokine | Redmond, William L. et al. (2012) PLoS ONE 7.4 Ed. Naglaa H. Shoukry. |
| | | | | Ruby et al., (2008) J Immunol. 180:2140-2148 |
| GITRL + tumor vaccine | | 1. EP of GITRL gene + cytotoxic agent (separately) to create local tumor antigen pool | System delivery of agonist Ab + alphavirus replicon particles encoding melanoma antigen | Avogadri et al.,_Cancer Immunol. Res. 2014 May;2(5):448-58 |
| | | | | Vergati et al., 2010. J. Biomed. Biotechnol. 2010:Article ID 596432 |
| | | 2. EP of GITRL gene + system delivery of tumor vaccine (i.e gp100 peptide vaccine for melanoma) | | |
| GITRL + Bleomycin, Gemzar, Cytozan, 5-fluoro-uracil, Adriamycin or other chemotherapeutic agent | | 1. intratumoral EP of drug + plasmid encoding GITRL | Intra-peritoneal injection of drugs + agonist Ab | US 8,591,886 |
| | | 2. EP of GITRL + system delivery of drug | | |
| GITRL + small molecule inhibitors (i.e. Sunitiinib, Imatinib, Vemurafenib, Trastuzumab, Bevacizumab, Cetuximb, rapamycin, Bortezomib, PI3K-AKT inhibitors, IAP inhibitors | | 1. EP of GITRL gene combined with local drug delivery | System ic delivery of therapeutic antibodies with targeted therapy drugs | Hu-Lieskovan et al.. (2014) J. Clin. Oncol. 32(21):2248-54 |
| | | 2. EP of GITRL gene combined with systemic drug treatment | | Vanneman and Dranoff (2014) Nat. Rev. Cancer 12(4): 237-251 |
| GITRL + targeted radiation | Sublethal radiation dose locally at tumor site, followed by GITRL EP | | Review outlining potential of radiation therapy in combination with immunotherapy, particularly T cell co-stimulation and/or checkpoint blockade | Almo SC, Guha C. (2014) Radiation Res. 182(2):230-238. |
| GITRL + Anti-PD1 antagonist Ab (+chemotherapeutic drugs) | EP of GITRL plus systemic anti-PD-1 Ab treatment | | Intra-peritoneal injection of therapeutic antibodies | Lu et al. (2014) J. Transl. Med. 12:36 |
| GITRL + anti-PDL1 antagonist Ab | EP of GITRL plus systemic anti-PDL-1 Ab treatment | | System ic treatment with blocking anti PDL-1 Ab + agonistic anti-CD137 Ab | Vezys et al., (2011) J Immunol. 187(4):1634-42 |

Other contemplated combination therapies are co-stimulatory agonists combined with: TLR agonists (e.g., Flagellin, CpG); IL-10 antagonists (e.g., anti-IL-10 or anti-IL-10R antibodies); TGFβ antagonists (e.g., anti-TGFβ antibodies); PGE2 inhibitors; Cbl-b (E3 ligase) inhibitors; CD3 agonists; telomerase antagonists, etc. In particular, various combinations of IL-12, IL-15/IL-15Ra, and/or GITR-L are contemplated. IL-12 and IL-15 have been shown to have synergistic anti-tumor effects (see, e.g., Kimura et al. (2000) Cancer Immunol. Immunother. 49:71-77). DNA encoding each molecule can be on separate expression plasmids or combined with appropriate promotors on a single expression plasmid, and delivered to the tumor by various techniques described below.

### Electroporation

The devices are contemplated for use in patients afflicted with cancer or other non-cancerous (benign) growths. These growths may manifest themselves as any of a lesion, polyp, neoplasm (e.g. papillary urothelial neoplasm), papilloma, malignancy, tumor (e.g. Klatskin tumor, hilar tumor, noninvasive papillary urothelial tumor, germ cell tumor, Ewing's tumor, Askin's tumor, primitive neuroectodermal tumor, Leydig cell tumor, Wilms' tumor, Sertoli cell tumor), sarcoma, carcinoma (e.g. squamous cell carcinoma, cloacogenic carcinoma, adenocarcinoma, adenosquamous carcinoma, cholangiocarcinoma, hepatocellular carcinoma, invasive papillary urothelial carcinoma, flat urothelial carcinoma), lump, or any other type of cancerous or non-cancerous growth. Tumors treated with the plasmids for use in the present embodiment may be any of noninvasive, invasive, superficial, papillary, flat, metastatic, localized, unicentric, multicentric, low grade, and high grade.

The devices are contemplated for use in numerous types of malignant tumors (i.e. cancer) and benign tumors. For example, the devices and methods described herein are contemplated for use in adrenal cortical cancer, anal cancer, bile duct cancer (e.g. periphilar cancer, distal bile duct cancer, intrahepatic bile duct cancer) bladder cancer, benign and cancerous bone cancer (e.g. osteoma, osteoid osteoma, osteoblastoma, osteochrondroma, hemangioma, chondromyxoid fibroma, osteosarcoma, chondrosarcoma, fibrosarcoma, malignant fibrous histiocytoma, giant cell tumor of the bone, chordoma, lymphoma, multiple myeloma), brain and central nervous system cancer (e.g. meningioma, astocytoma, oligodendrogliomas, ependymoma, gliomas, medulloblastoma, ganglioglioma, Schwannoma, germinoma, craniopharyngioma), breast cancer (e.g. ductal carcinoma in situ, infiltrating ductal carcinoma, infiltrating lobular carcinoma, lobular carcinoma in situ, gynecomastia), Castleman disease (e.g. giant lymph node hyperplasia, angiofollicular lymph node hyperplasia), cervical cancer, colorectal cancer, endometrial cancer (e.g. endometrial adenocarcinoma, adenocanthoma, papillary serous adnocarcinoma, clear cell) esophagus cancer, gallbladder cancer (mucinous adenocarcinoma, small cell carcinoma), gastrointestinal carcinoid tumors (e.g. choriocarcinoma, chorioadenoma destruens), Hodgkin's disease, non-Hodgkin's lymphoma, Kaposi's sarcoma, kidney cancer (e.g. renal cell cancer), laryngeal and hypopharyngeal cancer, liver cancer (e.g. hemangioma, hepatic adenoma, focal nodular hyperplasia, hepatocellular carcinoma), lung cancer (e.g. small cell lung cancer, non-small cell lung cancer), mesothelioma, plasmacytoma, nasal cavity and paranasal sinus cancer (e.g. esthesioneuroblastoma, midline granuloma), nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma (e.g. embryonal rhabdomyosarcoma, alveolar rhabdomyosarcoma, pleomorphic rhabdomyosarcoma), salivary gland cancer, skin cancer, both melanoma and non-melanoma skin cancer), stomach cancer, testicular cancer (e.g. seminoma, nonseminoma germ cell cancer), thymus cancer, thyroid cancer (e.g. follicular carcinoma, anaplastic carcinoma, poorly differentiated carcinoma, medullary thyroid carcinoma, thyroid lymphoma), vaginal cancer, vulvar cancer, and uterine cancer (e.g. uterine leiomyosarcoma).

The devices and methods described herein work to treat cancerous tumors by delivering electrical therapy continuously and/or in pulses for a period of time ranging from a fraction of a second to several days, weeks, and/or months to tumors. In a preferred embodiment, electrical therapy is direct current electrical therapy.

The term "electroporation" (i.e. rendering cellular membranes permeable) as used herein may be caused by any amount of coulombs, voltage, and/or current delivered to a patient in any period of time sufficient to open holes in cellular membranes (e.g. to allow diffusion of molecules such as pharmaceuticals, solutions, genes, and other agents into a viable cell).

Delivering electrical therapy to tissue causes a series of biological and electrochemical reactions. At a high enough voltage, cellular structures and cellular metabolism are severely disturbed by the application of electrical therapy. Although both cancerous and non-cancerous cells are destroyed at certain levels of electrical therapy tumor cells are more sensitive to changes in their microenvironment than are non-cancerous cells. Distributions of macroelements and microelements are changed as a result of electrical therapy.

In a single electrode configuration, voltage may be applied for fractions of seconds to hours between a lead electrode and the generator housing, to begin destruction of cancerous tissue. Application of a given voltage may be in a series of pulses, with each pulse lasting fractions of a second to several minutes. Low voltage may also be applied for of a duration of fractions of seconds to minutes, which may attract white blood cells to the tumor site. In this way, the cell mediated immune system may remove dead tumor cells and may develop antibodies against tumor cells. Furthermore, the stimulated immune system may attack borderline tumor cells and metastases.

Various adjuvants may be used to increase any immunological response, depending on the host species, including but not limited to Freund's adjuvant (complete and incomplete), mineral salts such as aluminum hydroxide or aluminum phosphate, various cytokines, surface active substances such as lysolecithin, pluronic polyols, polyanions, peptides, oil emulsions, and potentially useful human adjuvants such as BCG (bacille Calmette-Guerin) and *Corynebacterium parvum.* Alternatively, the immune response could be enhanced by combination and or coupling with molecules such as keyhole limpet hemocyanin, tetanus toxoid, diptheria toxoid, ovalbumin, cholera toxin or fragments thereof.

U.S. Patent No. 7,245,963 by Draghia-Akli, et al. describes modular electrode systems and their use for facilitating the introduction of a biomolecule into cells of a selected tissue in a body or plant. The modular electrode systems comprise a plurality of needle electrodes; a hypodermic needle; an electrical connector that provides a conductive link from a programmable constant-current pulse controller to the plurality of needle electrodes; and a power source. An operator can grasp the plurality of needle electrodes that are mounted on a support structure and firmly insert them into the selected tissue in a body or plant. The biomolecules are then delivered via the hypodermic needle into the selected tissue. The programmable constant-current pulse controller is activated and constant-current electrical pulse is applied to the plurality of needle electrodes. The applied constant-current electrical pulse facilitates the introduction of the biomolecule into the cell between the plurality of electrodes.

U.S. Patent Pub. 2005/0052630 describes an electroporation device which may be used to effectively facilitate the introduction of a biomolecule into cells of a selected tissue in a body or plant. The electroporation device comprises an electro-kinetic device ("EKD device") whose operation is specified by software or firmware. The EKD device produces a series of programmable constant-current pulse patterns between electrodes in an array based on user control and input of the pulse parameters, and allows the storage and acquisition of current waveform data. The electroporation device also comprises a replaceable electrode disk having an array of needle electrodes, a central injection channel for an injection needle, and a removable guide disk.

The electrode arrays and methods described in U.S. Patent No. 7,245,963 and U.S. Patent Pub. 2005/0052630 are adapted for deep penetration into not only tissues such as muscle, but also other tissues or organs. Because of the configuration of the electrode array, the injection needle (to deliver the biomolecule of choice) is also inserted completely into the target organ, and the injection is administered perpendicular to the target issue, in the area that is pre-delineated by the electrodes.

Typically, the electric fields needed for in vivo cell electroporation are generally similar in magnitude to the fields required for cells in vitro. The magnitude of the electric field may range from approximately, 10 V/cm to about 1500 V/cm, preferably from about 300 V/cm to 1500 V/cm and preferably from about 1000 V/cm to 1500 V/cm. Alternatively, lower field strengths (from about 10 V/cm to 100 V/cm, and more preferably from about 25 V/cm to 75 V/cm) the pulse length is long. For example, when the nominal electric field is about 25-75 V/cm, it is preferred that the pulse length is about 10 msec. The electroporation therapy for use in the invention comprises the administration of at least one voltage pulse of 200 V/cm to 1500 V/cm over a pulse width of 100 microseconds to 20 milliseconds.

The pulse length can be about 10 µs to about 100 ms. There can be any desired number of pulses, typically one to 100 pulses per second. The delay between pulses sets can be any desired time, such as one second. The waveform, electric field strength and pulse duration may also depend upon the type of cells and the type of molecules that are to enter the cells via electroporation. The electroporation therapy for use in the invention comprises the administration of at least one voltage pulse of 200 V/cm to 1500 V/cm over a pulse width of 100 microseconds to 20 milliseconds.

Uptake of the non-viral delivery vectors for use in the present invention may also be enhanced by plasma electroporation, also termed avalanche transfection. Briefly, microsecond discharges create cavitation microbubbles at electrode surface. The mechanical force created by the collapsing microbubbles combined with the magnetic field serve to increase transport efficiency across the cell membrane as compared with the diffusion mediated transport associated with conventional electroporation. The technique of plasma electroporation is described in Vankov, et al. United States Patent No. 7,923,251 issued April 12, 2011 and Vankov, et al United States Patent No 8,283,171 issued October 9, 2012. This technique may also be employed in vivo for the transformation of cells. Chalberg, et al (2006) Investigative Ophthalmology & Visual Science 47:4083-4090; Chalberg, et al United States Patent No 8,101169 Issued January 24, 2012.

### EXAMPLES

### I. Tumors and Mice

CT-26.WT cells obtained from ATCC (CRL2638) were thawed, and minimally passaged prior to implantation. Adherent CT-26.WT cells were removed from the plate with trypsin and cell viability was determined by AO/PI staining, and live/dead counting was performed using a Cellometer Auto2000 (Nexelcom Biosciences). Cells were resuspended in DPBS at a density of either 1.0 x 10⁷ live cells/ml or 0.5 x 10⁷/ml and kept on ice until implantation.

6 week-old female BALB/cJ (stock number 000651) mice purchased from Jackson Laboratories were shaved on both hind flanks one day prior to tumor implantation. Mice were anesthetized with isoflurane until unresponsive to a to pinch test. CT-26.WT cells were drawn into a 1.0 ml sterile syringe using an 18 Ga needle, which was replaced with a 26 Ga needle for implantation. CT-26.WT cell were injected subcutaneously, rostral to the hind flank in a volume of 0.1 ml resulting in implantation of 1.0 × 10⁶ cells (for primary tumor) or 0.5 × 10⁶ cells (for contralateral tumor).

Tumor growth was monitored starting 4 days after implantation by measuring long and short axes of tumors with digital calipers. Tumor volume was calculated using the formula for estimating ellipsoid volume (A² X B)/2, where "A" is the short axis and "B" is the long axis. Mice bearing bi-lobed tumors, or tumors implanted deeper than subcutaneous were discarded. When primary tumor volumes reached 40-90 mm³, mice were randomized into cohorts containing similar average tumor volumes. Mice are housed in accordance with AALAM guidelines.

### II. Plasmid DNA

pUMVC3 plasmids (ALDEVERON) for expression of both wild type, as well as recombinant soluble and membrane-bound forms of human GITRL, OX40L, or 4-1BB (structures illustrated in Figure 1) were generated. The extracellular domain (ECD) for each ligand was added (Genbank ID AF125303 [GITRL], D90224 [OX40L], and U03398 [4-1BBL].) In one variation (denoted GITRL4, OX40L4 and 4-1BBL4), a heterologous trimerization domain is added (Harbury, P.B. Nature 371: 80.) In another variation (denoted single chain trimer, SCT), 3 ECD domains were added in sequence, with linkers sequence composed of varying combinations of Glycine and Serine to introduce 'wobble'. The linker length varies between 12 and 23 amino acids. In some constructs the transmembrane domain from the PDGF receptor (taken from the vector pDisplay, Thermo Fisher) was added C-terminal to the ECD(s). In some constructs, the constant region (Fc) from human or mouse antibodies were added C terminal to the ECDs (human Fc-IgG1 [from pFUSE-hIgG1-Fc, InvivoGen], mouse Fc-IgG1 [from pFUSE-mIgG1-Fc, InvivoGen], mouse Fc-IgG2a [from pFUSE-mlgG2a-Fc, InvivoGen].)

Mouse and human GITRL are reported not to cross-react (see, e.g., Bossen et al., (2006) J. Biol. Chem. 281:13964-13971), thus mouse homologs for recombinant human GITRL were constructed for preclinical studies in mice. Mouse GITRL is naturally a dimer. Recombinant mouse GITRL constructs were made as both dimers and trimers using GCN4 multimerization motifs (Harbury, P.B. Nature 371: 80.) The relative binding of dimer and trimer forms to mouse GITR-Fc was compared using a functional ELISA. The trimer form showed higher binding affinity and was used for all further studies (EC50 for dimer, 39.24 uM; EC50 for trimer, 12.38 uM). Substituting the mouse GITRL ECD domain (Genbank Accession #NM_183391) for the human ECD domains in all constructs described above made mouse versions of the SCT forms used in pre-clinical studies. pUMVC3 containing a co-stimulatory molecule agonist (i.e. GITRL) is prepared with an endotoxin-free kit. All plasmid DNA was diluted in sterile injectable saline (0.9%) and stored at -20°C.

### III. Expression in tissue culture cells

Transfection of GITRL plasmids into HEK293 (ATCC) cells with Mirus TransIT-LT1 reagent (Cat.#: MIR 2300). Cell to be transduced are seated at 400,000 cells in a 6-well dish. 24 hours later, Cells were overlaid with 4µL Mirus and 1ug DNA). Cell were harvested 4-7 days later for analysis by Western blot, flow cytometry, For activity assays, secreted proteins were harvested from cellular supernatants, and purified using Ni⁺-resin according to the manufacturers instruction (Novagen)

### IV. Protein Detection by Western Blots and Flow Cytometry

For Western Blotting, laemmli SDS sample buffer (Alfa Aesar J61337) was added to each sample and boiled at 100 C for 10 minutes and samples were centrifuged. 20 µl of protein + buffer was loaded per well and gel was run at 150 volts for about an hour until the smallest standard reached the bottom of the gel. Gel proteins were transferred to PVDF membranes at 100 volts for 1 hour on ice, rinsed with PVDF 3X with 1X TBST, and then blocked for 1 hour at room temperature on a rocker with 5% BSA in TBST. Rinsed membranes were incubated overnight with Anti-NWSHPQFEK Tag antibody, mAb, mouse (Genscript A01732-100) diluted in TBST+5% nonfat dry milk. Blots were incubated for 1 hour at room temperature with 700 labeled anti-mouse secondary antibody (Rockland). Images were analyzed using a LICOR imager.

### V. Flow Cytometry

For flow cytometry, cells were removed from the dish using warm PBS without Ca++ or Mg++. Cells were counted and distributed into tubes with 5 × 10⁶ cells/tube, washed and resuspended in 100-200 µl FC buffer (5% filtered FBS + 0.1% NaN₃ in PBS without Ca++ or Mg++). PE-labeled anti-GITRL (clone 109101 R&D systems), isotype control (clone 11711 R&D systems), or anti-FC and corresponding isotype control (Biolegend) were added and incubated on ice for 1 hour. Samples were washed 3 times with FC buffer. In the case where the sample was to be incubated with anti-FC, the cells were first incubated with anti-GITR-FC fusion protein in FC buffer for 1 hour on ice. Cells were analyzed using a Becton-Dickenson FACScan, GUAVA 12HT flow cytometer (Millipore), or LSR-II (Beckman).

When transduced into HEK 293 human cells, proteins from these constructs were synthesized and properly localized to the extracellular space (soluble form) or on the cell surface and detectable with Anti-NWSHPQFEK Tag antibody by Western Blot, and anti-GITRL Ab by flow cytometry. Membrane-bound trimerized recombinant GITRL proteins, when expressed in HEK 293 cells bound to soluble GITR-FC fusion proteins.

**Table 2: Mean Fluorescence Intensities of transfected HEK 293 cells expressing cell surface GITR proteins demonstrating cell surface expression and binding to soluble GITR-Fc fusion protein (R&D Systems)**

| **Protein** | **Anti-GITRL** | **GITR-Fc + Anti-Fc** | **Anti-Fc** |
|---|---|---|---|
| TM1 | 159.5 | 192.9 | 13.5 |
| TM-SCT | 982.5 | 771.4 | 13.0 |

### VI. Receptor Binding by functional ELISA

Anti human-Fc antibody (Pierce 31125) was added to flat-bottomed 96 well ELISA plates (Costar cat# 3690), diluted to 1 µg/ml diluted with PBS, and incubated at room temperature for 1 hour. Wells were washed with 150 µl PBST 3X, decanted and dried. Wells were blocked by adding 150 µl/well Superblock (Scytek AAA999) and incubated at room temperature for 1 hr. After washing, 50 µl/well of recombinant human GITR/TNFRSF18 Fc chimeric protein (R&D 689-GR-100) was added at a concentration of 500 ng/ml. Samples were incubated at room temperature for 1 hour, and subsequently washed. The following were added in triplicate: 50 µl of 5000 ng/ml rhGITRL standard Recombinant Human GITR Ligand/TNFSF18 (R&D 6987-GL-025) diluted fivefold with PBS down to zero; 50 µl of neat HEK 293 culture supernatant (negative control with no gene expression) diluted fivefold with PBS down to zero; and the soluble human GITRL protein, also titrated. Samples were incubated at room temperature for 1 hour and then washed again. 50 µl/well mouse anti human biotinylated hGTIRL antibody (R&D BAM6943) at 500 ng/ml diluted with PBS was added to each well and incubated at room temperature for 1 hour. After washing, 50 µl/well of 1:15000 strepavidin HRP (Abcam 7403) was added. Samples were incubated at room temperature for 1 hour. After washing, 50 ul/well TMB substrate (Pierce cat# 34028) was added and samples were incubated for 10 minutes. Reaction was stopped with the addition of 25 µl/well H₂SO₄. Optical density was determined using microplate reader set to 450 nm, with wavelength correction set to 540 or 570 nm. The concentration of GITRL in culture supernatants was determined by conventional ELISA using anti-GITRL antibody for binding and detection, and a GITRL protein standard (R&D 6987-GL-025)

Soluble GITRL4 and GITRL-SCT-Fc proteins bind to GITR-FC fusion protein in an ELISA with greater than a 2-fold higher affinity than that of commercially available GITRL protein ((R&D 6987-GL-025; Table 3), and a greater than 2-fold higher specific activity (activity units per ng; Figure 2).. Soluble recombinant GITRL proteins demonstrated comparable GITR binding affinity to cross-linked MK-4166.

**Table 3: Soluble GITRL protein binding affinity to plate-bound GITR-Fc fusion protein and comparison with cross-linked MK-4166 GITR agonist antibody (see, e.g, US8709424).**

| Protein | R&D GITRL | GITRL4 | GITRL-SCT | GITRL-SCT-Fc | MK-4166 |
|---|---|---|---|---|---|
| EC50 | 1.495 | 0.5806 | 4.759 | 0.5355 | 0.2912 |

### VII. NFkB-luciferase Reporter Assays

GITRL plasmids were transduced into HEK 293 human cells together with plasmids encoding firefly luciferase under the control of an NFkB-driven promoter (Promega). The plasmid expressing the human GITR gene (Origene), and a plasmid expressing Renilla luciferase were under the control of a constitutive CMV promoter (Promega; control for transduction variability). After 48 hours after transfection, cells were lysed and analyzed for both firefly and Renilla luciferase activity using a Dual Luciferase assay kit (Promega, E1910). Soluble GITRL proteins stimulated GITR signaling to NFkB in a reporter cell line system (Figure 4).
Soluble GITRL proteins were purified from HEK 293 culture supernatants in serum-free conditions using Ni+-Resin as per the manufactures instructions (Novagen). Purified proteins were normalized by A280 absorbance and by ELISA. Molar equivalents of protein were added to engineered Jurkat cells expressing human GITR and an NF-kB-driven secreted luciferase (GITR potency assay, Promega CS184002). A dose response was measured for each protein and compared with commercially available soluble human GITRL (R&D systems). Assay was carried out as per the manufactured instruction with and without the addition of cross-linking antibodies: Anti-HA for R&D GITRL standard (R&D Systems) and Anti-NWSHPQFEK Tag (Genscript) for our GITRL proteins.

Cell surface GITRL proteins were also tested in the GITR potency assay (Promega CS184002) by adapting the assay protocol to co-culture stimulation. HEK 293 cells were transduced with plasmids encoding cell surface GITRL proteins were removed from the dish with PBS-Mg++-Ca++ after 4 days in culture, and plated in a serial dilution from 100,000 down to 1 cell/well in flat bottom 96-well tissue-culture trays (Corning) and left overnight to adhere to substrate. Thaw and use Jurkat cells (Promega CS184002) were overlaid according to the manufacturer instruction onto GITRL-expressing HEK 293 cells and co-cultured for 7 hours. Supernatants were removed from co-culture and luciferase activity was measured as described in the standard protocol (Promega GITR Potency Assay) using a BioTek luminometer. Results were compared with untranfected cells as a negative control, and with soluble R&D GITRL as a positive control for each experiment.

**Table 4: Soluble GITRL proteins show strong activation of GITR in Promega luciferase reporter assay**

| **Protein** | **EC50 (uM)** | |
|---|---|---|
| | **- antibody** | **+ antibody** |
| hGITRL4 | 0.31 | 0.10 |
| hGITRL-SCT | 39.10 | 2.26 |
| hGITRL-SCT-Fc | 0.29 | 0.28 |
| R&D hGITRL | | 0.97 |

Soluble GITRL4 and GITRL-SCT-FC had greater than 3-fold and 10-fold better potency in this GITR activity assay, respectively, than did commercially available human GITRL (R&D Systems). Cell surface GITRL also showed strong activation when co-cultured with Jurkat reporter cells in GITR potency assay (Figure 4).

### VIII. Primary human T cell stimulation and cell surface binding of soluble GITRL

Primary human Pan T cells (Allcells, PB009-1) were plated in wells coated overnight with anti-CD3 (OKT3 10 ug/ml) alone or in combination with soluble anti-CD28 (15E8, I ug/ml) or culture supernatants from HEK 293 cells with and without prior transfection with soluble GITRL. Cell were cultured in RPMI + 10% FBS for 4 days. Un-stimulated control cells were plated in RPMI + 10% FBS in parallel.

For cell surface binding, cells that had been stimulated with anti-CD3 and anti-CD28 were washed with FC buffer (5% filtered FBS + 0.1% NaN3 in PBS without magnesium or calcium), on ice. Cells were incubated with or without HEK 293 culture supernatants for 1 hour on ice. After washing in FC buffer, cells were incubated with conjugated antibodies: FITC-conjugated-StrepTAG II antibody (GenScript A10736-100), FITC-conjugated isotype control (Invitrogen GM4992), PE-conjugated human GITR antibody. Cells are washed with FC buffer and analyzed by flow cytometry as described above.

For proliferation assay, duplicate wells of stimulated cells or un-stimulated controls were stained with AO/PI vital staining solution (Nexcelom CS2-0106-5ML), and counted on a Cellometer Auto 2000 4 days after stimulation.

For cytokine production assays, CD4+ T-cells and Pan T-cells (Allcells) were costimulated with 0.1, 0.2, or 0.5 ug/ml of anti-human CD3 (Cat #: 100207) from Biolegend and soluble agonist GITRL molecules. Anti-human CD28 (Cat #: 102111) was used as a positive control. Costimulated T-cells were incubated for 72 hours at 37°C before supernatants were collected. The level of human IFN-y and IL-2 in cell supernatants was measured by R&D ELISA kits (Cat #: DY485-05 and Cat #: DY402-05).

Recombinant soluble GITRL4 bound to the cell surface of stimulated primary human pan T cells.

**Table 5. Mean Fluorescence intensities (MFI) are shown for T cells stained with an antibody against the epitope tagged-GITRL4 protein after incubation with the culture supernatants from pUMVC3-GITRL4 transduced cells as compared witih isotype controls or incubation of T cells with supernatants from untransduced cells.**

| Pre-incubation | Mean fluorescence intensity | |
|---|---|---|
| (-) | Isotype control-PE | Anti-GITR-PE |
| | 6.10 | 55.18 |
| GITRL4 culture supernatant | Isotype control-FITC | Anti-StrepTagII-FITC |
| | 5.08 | 48.40 |
| Control culture supernatant | Isotype control-AF488 | Anti-StrepTagII-AF488 |
| | 4.90 | 8.51 |

Addition of culture supernatant from HEK 293 cells transduced with plasmids expressing GITRL4 increase primary human T cell proliferative response to TCR crosslinking with anti-CD3 antibody,, but to a smaller extent than anti-CD28 antibodies. Addition of GTRL proteins also increase cytokine production in response to TRC cross-linking,

**Table 6: Stimulation of Primary T cell proliferation by GITRL4 protein. The concentration of cells are shown per milliliter in millions after stimulation with the indicated proteins.**

| **Biological duplicates** | **No stimulation** | **Anti-CD3** | **Anti-CD3 + GITRL sup** | **Anti-CD3 + control sup** | **Anti-CD3 + Anti-CD28** |
|---|---|---|---|---|---|
| Well 1 | 1.83 | 3.01 | 3.42 | 2.45 | 3.89 |
| Well 2 | 2.16 | 2.55 | 3.32 | 2.7 | 3.93 |

**Table 7: When INFy levels were measured in the culture supernatants from primary human Pan T cells, an increase in this cytokine was seen when soluble GITRL4 protein was added along with suboptimal levels of Anti-CD3 (OKT3) antibody. Each value represents the average of triplicate wells of stimulated T cell supernatants.**

| **Protein (ug/ml)** | **IFN-γ (pg/ml)** | |
|---|---|---|
| | **0.2 OKT3** | **0.5 OKT3** |
| none | 235 | 1005 |
| α-CD28 | 6824 | 10079 |
| 0.2 R&D hGITRL | 302 | 846 |
| 1.0 R&D hGITRL | 2006 | 3273 |
| 0.2 hGITRL4 | 361 | 839 |
| 1.0 hGITRL4 | 2821 | 4425 |

**Table 8: Fc-SCT GITRL proteins increased production of INFγ and IL-2 from CD4+ primary human T cells stimulated with sub-optimal doses of anti-CD3.**

| **Protein (µM)** | **IFN-γ (ng/ml)** | | **IL-2 (ng/ml)** | |
|---|---|---|---|---|
| | **0.1 OKT3** | **0.5 OKT3** | **0.1 OKT3** | **0.5 OKT3** |
| unstimulated | 0.021 | 0.007 | 0.025 | 0.007 |
| none | 0.066 | 0.313 | 0.032 | 0.043 |
| 15 a-CD28 | 1.764 | 3.180 | 0.402 | 0.734 |
| 100 hGITRL-SCT-Fc | 0.115 | 0.777 | 0.041 | 0.127 |
| 10 hGITRL-SCT-Fc | 0.131 | 0.734 | 0.043 | 0.099 |

Collectively, these data indicate that plasmids encoding the recombinant GITRL proteins are expressed when introduced into cells and can bind to and stimulate cell surface endogenous GITR on T cells. Within the tumor microenvironment, stimulation of cell surface GITR on T lymphocytes is predicted to enhance activation of these lymphocytes within the tumor microenvironment and facilitate an immune response against tumor cells.

### IX. Comparison of mouse homologs for recombinant GITRL with DTA-1 in primary mouse T cells.

Mouse recombinant GITRL proteins were expressed in HEK 293 cells and the retention in the cell (those containing TM domains) or secretion into the culture medium was verified by Western Blot. Binding to GITR on the surface of primary mouse splenocytes was verified by flow cytometry using anti-StrepTAG II antibody (Genscript). Further, an increase in stimulation of primary mouse T cells with a sub-optimal dose of anti-CD3 (1452C11) with addition of purified soluble mouse GITRL proteins was observed in both purified CD4+ and CD8+ T cells, and well as in whole splenocytes cultures. In each case, recombinant mouse GITRL showed comparable or better T cell co-stimulatory activity than the GITR agonist antibody, DTA-1 (BXcell BE0063). For example, in whole splenocytes plated with 0.5 ugs/ml 1452C11 anti CD-3 Ab (Biolegend 100207), DTA-1 had an EC50 of 1.29 uM, mGITRL-SCT-FcIgG2a had an EC50 of 0.473 uM, mGITRL4 had an EC50 of 3.32 uM.

### X. Intratumoral Treatment

Mice were anesthetized with isoflurane for treatment. Circular plasmid DNA was diluted to 1 ug/ul in sterile 0.9% saline. 50 ul of plasmid DNA was injected centrally into primary tumors using a 1 ml syringe with a 26 Ga needle. Electroporation was performed immediately after injection. Electroporation of DNA was achieved using a BTX ECM 830 square wave electroporator providing 8 pulses of 350 V/cm, 10 msec each spaced 1 second apart. Electroporation was delivered by a Medpulser handle with needles spaced 0.5 cm apart, configured to deliver current uni-directionally from 2 needles to 2 opposing needles. The electroporation needles were inserted into the tumor where tumor size permitted. Treatments were performed on days 0,4, and 7, or days 0 and 4 depending on the study. Following treatment, tumor volume was determined every 2-3 days using digital calipers as described previously. Mice were euthanized when the volume of either the primary or contralateral tumor reached 1000 mm³.

For each experiment, 10-15 mice cohort were electroporated with plasmids encoding recombinant mouse GITRL proteins and compared with a cohort treated i.p with 500 ugs DTA-1 GITR agonist antibody on Day 1 or left untreated.

As compared with untreated mice, tumor volumes for the electroporated tumors were significantly reduced (Figure 5A). Moreover, statistically significant reduction was also observed in untreated tumors on the contralateral flank of the mouse (Figure 5B). Treated tumors demonstrated comparable reduction in volume as systemic DTA-1 treatment.

### XI. Characterization of T cells in the spleen and tumor by flow cytometry

Tumors and spleen were excised from sacrificed mice and placed in RPMI + 10% FBS. Splenocytes were isolated by pressing spleen though a 70 micron strainer, subjecting them to hypotonic red blood cell lysis (ACK buffer; ThermoFisher). Isolate splenocytes were purified using lympholyte M (Cedarlane) prior to staining. Tumors were dissociated using Gentle-MACS for tumors (Miltenyi tumor dissociation kit 130-096-730, C-tubes, 130-093-237) and homogenized using an Miltenyi gentleMACS^{™} Octo Dissociator with Heaters (130-096-427). Samples were stored on ice until all samples were ready. Cells were pelleted at 1200 rpm (800 x g) for 5 min at 4C and resuspended in PBS + 2% FBS + 1 mM EDTA (PFB) and overlaid onto 5 mL of Lympholyte-M (Cedarlane) in 15-mL conical centrifuge tubes. Lympholyte columns were spun in centrifuge at 2000 rpm (1500 x g) for 20 min at room temperature with no brake. Lymphocyte layer was transferred to 10 mL of PBF, then centrifuge 1200 rpm (800 x g) for 5 min at 4C. Cell pellets were gently resuspended in 500 uL of PFB with Fc block (BD Biosciences 553142). In 96-well plate, cells were mixed with a solution of AH1-detramer (immudex JG3294-APC), according to the manufacturers instruction and incubated for 10 minutes at room temperature. Antibody staining cocktails containing the following: Anti-CD45-AF488 (Biolegend 100723), anti-CD3-BV785 (Biolegend 100232), Anti-CD4-PE (eBioscience12-0041), anti-CD8a-APC (eBioscience 17-0081), anti-CD44-APC-Cy7 (Biolegend 103028), anti-CD19-BV711 (Biolegend 11555), were added and incubated at room temperature for 30 minutes. Cells were washed 3 times and fixed in PFB with 1% paraformaldehyde for 15 minutes on ice. Cells were washed with PFB and stored at 4'C in the dark. Samples were analyzed on an LSR II flow cytometer (Beckman).

Intratumoral electroporation of GITRL increased AH1-dextramer - binding CD8+CD44+ T cells in the spleen (Figure 6). An increase in activated CD8+ effector T cells that bind to the AH1 peptide, representing the immunodominant antigen in CT26 tumors, indicates an increase in systemic immunity to CT26 tumors in treated mice.

RT-PCR was used to measure gene expression changes within the treated tumor.. Flash frozen tumors were resuspended in PBS and homogenized using gentle MACS Dissociator (Miltenyl Biotech). The homogenate was then transferred into Trizol (Life Technologies Corp.). Total RNA was isolated according to manufacturer's protocol followed by DNase treatment. 1ug RNA was used to prepare cDNA (Maxima H Minus First Strand cDNA Synthesis Kit with dsDNase, Thermo Fisher Scientific). RT-PCR was performed using TaqMan^{®} Fast Advanced Master Mix (Thermo Fisher Scientific.) and a CFX96 (Biorad). Relative mRNA levels were normalized to 18s and the cycle number was used to calculate the amount of each product using the 2-ΔΔCT method (Livak et al, Methods, 2001).

Intratumoral electroporation of GITRL decreased Foxp3 gene expression within the treated tumor (Figure 7). Expression of the Foxp3 transcription factor is a marker for regulatory T cells (Treg), which perform an immunosuppressive function within the tumor microenvironment. DTA-1, a GITR agonist antibody is known to reduce the number of Tregs within tumors, and has also been shown to reduce the levels of Foxp3 gene present in tumors (Schaer et al., (2013) Cancer Immunol Res. 1:320-331) Likewise, we observed a reduction in Foxp3 gene levels in tumor electroporated with pUMVC3-GITR4 as compared with tumors elecroporated with pUMVC3 empty vector.

These analyses of T cells from spleens and tumors of treated mice suggest that intratumoral electroporation of plasmid encoding for recombinant GITRL can alter the Treg levels within the treated tumor, and result in an increase in tumor antigen-reactive effector T cells systemically.

In addition to GITRL monotherapy, a combination study was done using the CT26 syngeneic tumor model. pUMVC3 plasmid encoding GITRL4-TM1 was electroporated intratumorally along with pUMVC3 encoding for the p35 and p40 subunits of the IL-12 cytokine (A Daud et al., (2008) J. Clin. Oncol. 26:5896-5903).

A statistically significant decrease in mean tumor volume of the untreated, contralateral tumor was seen as compared with pUMVC3 vector control or pUMVC3-IL12 plasmid alone as measured 6, 8, 11 and 13 days after first EP treatment. In addition, complete tumor regression of the untreated tumors had faster kinetics. For example, 19 days after treatment began, 7/15 mice had completed regressed contralateral tumors while the IL-12 treated cohort had only 1/15. Moreover, an increase in the number of mice with a complete response (CR) on both the primary (treated) and contralateral (untreated) was observed as compared with pUMVC3-IL-12 alone.

**Table 8: Intratumoral electroporation of plasmids encoding recombinant GITRL and the cytokine, IL-12 reduced tumor growth. Contralateral tumor volume measured on day 11 is shown, as well as the incidence of complete tumor regression (CR), partial response (PR), and progressive disease (PD) in both treated and untreated tumors as measured 26 days after the first EP treatment are shown (P=Primary tumor; C = Contralateral tumor).**

| Treatment | tumor volume mm³ (mean+/- SEM) | Number of mice with CR out of 15 | | Number of mice with PR out of 15 | | Number of mice with PD out of 15 | |
|---|---|---|---|---|---|---|---|
| | C | P | C | P | C | P | C |
| Untreated | 751.7 +/-175.5 | 0 | 0 | 1 | 2 | 14 | 13 |
| pUMVC3 | 675.7 +/-36.5 | 2 | 0 | 1 | 3 | 12 | 12 |
| pUMVC3-IL-12 | 176.6 +/-115.8 | 13 | 8 | 0 | 3 | 2 | 5 |
| pUMVC3-GITRL-TM1/pUM VC3-IL-12 | 64.25 +/-61.8 | 13 | 11 | 0 | 1 | 2 | 3 |

These results indicate that combination therapy with GITRL and IL-12 in the tumor microenvironment of the primary (treated) tumor had greater efficacy than did IL-12 alone on an untreated, contralateral tumor.

### X. Intratumoral Treatment with IL-12, IL-15/IL15Ra, GITRL

IL-12, IL-15 and GITRL all have separate effect on stimulation of the immune system. Plasmids encoding IL-12 α and β subunits, IL-15 and IL-15Ra, and recombinant GITRL are electroporated together (on separate plasmids or in a single plasmid) into CT26 tumors in a contralateral tumor model to assess whether the combination of these 5 genes has more efficacy in regressing established tumors in BALB/c mice. Intratumoral electroporation of a single, large plasmid encoding all 5 genes is also tested for efficacy as compared with untreated mice. In addition, testing of these genes in combination and separately is done in a B16F10 contralateral tumor model in C57/Bl6 mice. Tumor volumes are measured over time, and the percent of mice with complete response are measured.

### XI. Histology

Mice are humanely sacrificed by C0₂ asphyxiation. Tumors are excised and placed in 50-ml conical tubes containing 10 ml of 10% formalin. The tissue is stained with H&E after fixation, as follows: after fixation in 10% neutral buffered formalin for 6 hours, representative tissue samples are processed into paraffin blocks using a Miles VIP tissue processor (Miles Inc., Mishawaka, IN). Briefly, tissues are dehydrated in ascending grades of ethanol, cleared in xylene, and infiltrated in paraffin (Tissue Prep 2; Fisher Scientific). Following embedding, tissues are sectioned on a standard rotatory microtome and 4 mm sections are retrieved from a waterbath and mounted on glass slides. Three sections per tumor are examined. Sections are heat-dried and stained with H&E (Richard-Allen Scientific, Kalamazoo, MI) using standard histologic techniques.

### XII. Immunohistochemistry

Immunohistochemical staining is conducted to examine the tumors for the presence of CD4+ lymphocytes, CD8+ lymphocytes, and blood vessels using the following antibodies: rat anti-mouse CD4, rat anti-mouse CD8 (Ly2), and rat anti-mouse CD31 (PECAM-1), respectively (PharMingen, Cambridge, MA). Mice are humanely sacrificed by C0₂ asphyxiation. Tumors are excised with scissors and the skin removed, then immediately frozen in a mixture of dry ice and ethanol, and stored at (80°C). Frozen sections of 5 m are obtained. For immunohistochemical analysis, rat anti-mouse CD4, rat anti-mouse CD8 (Ly2), or rat anti-mouse CD31 (PECAM-1) is applied to tissue sections at a dilution of 1:50 and incubated for 30 minutes, followed by detection with the Vector Elite Rat IgG Peroxidase kit at 2X concentration (15 minutes each in biotinylated anti-rat IgG and ABC complex). Immunostaining is carried out on the Dako autostainer. Sections are analyzed at 400X magnification.

### XIII. Statistical methods

Statistical analysis is performed by ANOVA, Mann-Whitney test, or two-tailed Student's T-test.

## Claims

1. A plasmid coding for a membrane bound form of an agonist of GITR, CD137, or CD134, wherein the agonist is a membrane bound form of GITR-L, CD137-L, CD134-L, for use in a method of treating a subject having a cancerous tumor, the method comprising: injecting the cancerous tumor with an effective dose of said plasmid and a nucleic acid encoding at least one immunostimulatory cytokine; and administering electroporation therapy to the tumor, the electroporation therapy comprising the administration of at least one voltage pulse of 200 V/cm to 1500 V/cm over a pulse width of 100 microseconds to 20 milliseconds.

2. The plasmid for use in the method of claim 1, wherein the cancerous tumor is melanoma.

3. The plasmid for use in the method of any one of claims 1-2, wherein the plasmid further comprises the nucleic acid encoding at least one immunostimulatory cytokine.

4. The plasmid for use in the method of any one of claims 1-3, wherein the immunostimulatory cytokine is selected from the group consisting of: IL-12, IL-15, and a combination of IL-12 and IL-15.

5. The plasmid for use in the method of any one of claims 1-4, wherein the nucleic acid encoding at least one immunostimulatory cytokine encodes an IL-12 p35 subunit and an IL-12 p40 subunit.

6. The plasmid for use in the method of any one of claims 1-5, wherein the method comprises: administering a first treatment at a first time (T1), wherein the first treatment comprises injecting the cancerous tumor with a first effective dose of said plasmid and administering a first electroporation therapy to the tumor at time T1, the first electroporation therapy comprising the administration of at least one voltage pulse having a duration of 100 microseconds to 20 milliseconds; and administering a second treatment at a second time (T2), wherein time T2 is a time later than time T1, wherein the second treatment comprises injecting the cancerous tumor with a second effective dose of said plasmid and administering a second electroporation therapy to the tumor at time T2, the second electroporation therapy comprising the administration of at least one voltage pulse having a duration of 100 microseconds to 20 milliseconds.

7. The plasmid for use in the method of claim 6, wherein the method further comprises: administering a third treatment at a third time (T3), wherein time T3 is a time later than time T2, wherein the third treatment comprises injecting the cancerous tumor with a third effective dose of said plasmid and administering a third electroporation therapy to the tumor, the third electroporation therapy comprising the administration of at least one voltage pulse having a duration of 100 microseconds to 20 milliseconds.

8. The plasmid for use in the method of claim 6, wherein the method further comprises: injecting an effective dose of said plasmid into the cancerous tumor of the subject; and administering electroporation to the subject intratumorally using at least one low voltage pulse having a pulse width of 100 microseconds to 20 milliseconds.

9. A plasmid coding for a membrane bound form of an agonist of GITR, CD137, or CD134, wherein the agonist is a membrane bound form of GITR-L, CD137-L, CD134-L, for use in a method of treating distant tumors and metastases in a subject having a cancer, the method comprising: injecting a cancerous tumor with an effective dose of said plasmid and a nucleic acid encoding at least one immunostimulatory cytokine; and administering electroporation therapy to the tumor, the electroporation therapy comprising the administration of at least one voltage pulse of 200 V/cm to 1500 V/cm over a pulse width of 100 microseconds to 20 milliseconds.

10. The plasmid for use in the method of claim 9, wherein the cancer is melanoma.

11. The plasmid for use in the method of claim 9 or 10, wherein the plasmid further comprises the nucleic acid encoding at least one immunostimulatory cytokine.

12. The plasmid for use in the method of any one of claims 9-11, wherein the immunostimulatory cytokine is selected from the group consisting of: IL-12, IL-15, and a combination of IL-12 and IL-15.

13. The plasmid for use in the method of any one of claims 9-12, wherein the nucleic acid encoding at least one immunostimulatory cytokine encodes an IL-12 p35 subunit and an IL-12 p40 subunit.

## Patentansprüche

1. Ein Plasmid, das für eine membrangebundene Form eines Agonisten von GITR, CD137 oder CD134 verschlüsselt, wobei der Agonist eine membrangebundene Form von GITR-L, CD137-L, CD134-L zur Verwendung bei einem Verfahren zur Behandlung einer Testperson mit einem krebsartigen Tumor ist, wobei das Verfahren Folgendes beinhaltet: Einspritzen des krebsartigen Tumors mit einer wirksamen Dosis des Plasmids und einer Nukleinsäure, die mindestens ein immunstimulierendes Cytokin codiert; und Verabreichen von Elektroporationstherapie an den Tumor, wobei die Elektroporationstherapie das Verabreichen von mindestens einem Spannungsimpuls von 200 V/cm bis 1500 V/cm über eine Impulsbreite von 100 Mikrosekunden bis 20 Mikrosekunden beinhaltet.

2. Plasmid zur Verwendung bei dem Verfahren gemäß Anspruch 1, wobei der krebsartige Tumor ein Melanom ist.

3. Plasmid zur Verwendung bei dem Verfahren gemäß einem der Ansprüche 1-2, wobei das Plasmid ferner die Nukleinsäure beinhaltet, die mindestens ein immunstimulierendes Cytokin codiert.

4. Plasmid zur Verwendung bei dem Verfahren gemäß einem der Ansprüche 1-3, wobei das immunstimulierende Cytokin aus der Gruppe ausgewählt ist, die aus Folgendem besteht: IL-12, IL-15 und einer Kombination von IL-12 und IL-15.

5. Plasmid zur Verwendung bei dem Verfahren gemäß einem der Ansprüche 1-4, wobei die Nukleinsäure, die mindestens ein immunstimulierendes Cytokin codiert, eine IL-12 p35-Untereinheit und eine IL-12 p40-Untereinheit codiert.

6. Plasmid zur Verwendung bei dem Verfahren gemäß einem der Ansprüche 1-5, wobei das Verfahren Folgendes beinhaltet: Verabreichen einer ersten Behandlung zu einer ersten Zeit (T1), wobei die erste Behandlung das Einspritzen des krebsartigen Tumors mit einer ersten wirksamen Dosis des Plasmids und das Verabreichen einer ersten Elektroporationstherapie an den Tumor zu einer Zeit T1 beinhaltet, wobei die erste Elektroporationstherapie das Verabreichen mindestens eines Spannungsimpulses mit einer Dauer von 100 Mikrosekunden bis 20 Mikrosekunden beinhaltet; und das Verabreichen einer zweiten Behandlung zu einer zweiten Zeit (T2), wobei die Zeit T2 eine Zeit später als Zeit T1 ist, wobei die zweite Behandlung das Einspritzen des krebsartigen Tumors mit einer zweiten wirksamen Dosis des Plasmids und das Verabreichen einer zweiten Elektroporationstherapie an den Tumor zu der Zeit T2 beinhaltet, wobei die zweite Elektroporationstherapie das Verabreichen von mindestens einem Spannungsimpuls mit einer Dauer von 100 Mikrosekunden bis 20 Millisekunden beinhaltet.

7. Plasmid zur Verwendung bei dem Verfahren gemäß Anspruch 6, wobei das Verfahren ferner Folgendes beinhaltet: Verabreichen einer dritten Behandlung zu einer dritten Zeit (T3), wobei die Zeit T3 eine Zeit später als die Zeit T2 ist, wobei die dritte Behandlung das Einspritzen des krebsartigen Tumors mit einer dritten wirksamen Dosis des Plasmids und das Verabreichen einer dritten Elektroporationstherapie an den Tumor beinhaltet, wobei die dritte Elektroporationstherapie die Verabreichung von mindestens einem Spannungsimpuls mit einer Dauer von 100 Mikrosekunden bis 20 Millisekunden beinhaltet.

8. Plasmid zur Verwendung bei dem Verfahren gemäß Anspruch 6, wobei das Verfahren ferner Folgendes beinhaltet: Einspritzen einer wirksamen Dosis des Plasmids in den krebsartigen Tumor der Testperson; und Verabreichen von Elektroporation an die Testperson unter intratumoraler Verwendung mindestens eines Impulses niedriger Spannung, der eine Impulsbreite von 100 Mikrosekunden bis 20 Millisekunden aufweist.

9. Plasmid, das für eine membrangebundene Form eines Agonisten von GITR, CD137 oder CD134 verschlüsselt, wobei der Agonist eine membrangebundene Form von GITR-L, CD137-L, CD134-L zur Verwendung bei einem Verfahren zur Behandlung von distanten Tumoren und Metastasen bei einer Testperson mit einem Krebs ist, wobei das Verfahren Folgendes beinhaltet: Einspritzen eines krebsartigen Tumors mit einer wirksamen Dosis des Plasmids und einer Nukleinsäure, die mindestens ein immunstimulierendes Cytokin codiert; und Verabreichen von Elektroporationstherapie an den Tumor, wobei die Elektroporationstherapie die Verabreichung von mindestens einem Spannungsimpuls von 200 V/cm bis 1500 V/cm über eine Impulsbreite von 100 Millisekunden bis 20 Millisekunden beinhaltet.

10. Plasmid zur Verwendung bei dem Verfahren gemäß Anspruch 9, wobei der Krebs ein Melanom ist.

11. Plasmid zur Verwendung bei dem Verfahren gemäß Anspruch 9 oder 10, wobei das Plasmid ferner die Nukleinsäure beinhaltet, die mindestens ein immunstimulierendes Cytokin codiert.

12. Plasmid zur Verwendung bei dem Verfahren gemäß einem der Ansprüche 9-11, wobei das immunstimulierende Cytokin aus der Gruppe ausgewählt ist, die aus Folgendem besteht: IL-12, IL-15 und einer Kombination von IL-12 und IL-15.

13. Plasmid zur Verwendung bei dem Verfahren gemäß einem der Ansprüche 9-12, wobei die Nukleinsäure, die mindestens ein immunstimulierendes Cytokin codiert, eine IL-12 p35-Untereinheit und eine IL-12 p40-Untereinheit codiert.

## Revendications

1. Un plasmide codant pour une forme liée à une membrane d'un agoniste de GITR, CD137, ou CD134, l'agoniste étant une forme liée à une membrane de GITR-L, CD137-L, CD134-L, pour une utilisation dans une méthode de traitement d'un sujet ayant une tumeur cancéreuse, la méthode comprenant : le fait d'injecter, dans la tumeur cancéreuse, une dose efficace dudit plasmide et un acide nucléique encodant au moins une cytokine immunostimulatrice ; et le fait d'administrer une thérapie par électroporation à la tumeur, la thérapie par électroporation comprenant l'administration d'au moins une impulsion de tension de 200 V/cm à 1 500 V/cm sur une largeur d'impulsion de 100 microsecondes à 20 millisecondes.

2. Le plasmide pour une utilisation dans la méthode de la revendication 1, où la tumeur cancéreuse est un mélanome.

3. Le plasmide pour une utilisation dans la méthode de n'importe laquelle des revendications 1 à 2, où le plasmide comprend en outre l'acide nucléique encodant au moins une cytokine immunostimulatrice.

4. Le plasmide pour une utilisation dans la méthode de n'importe laquelle des revendications 1 à 3, où la cytokine immunostimulatrice est sélectionnée dans le groupe constitué de : IL-12, IL-15, et d'une combinaison de IL-12 et IL-15.

5. Le plasmide pour une utilisation dans la méthode de n'importe laquelle des revendications 1 à 4, où l'acide nucléique encodant au moins une cytokine immunostimulatrice encode une sous-unité IL-12 p35 et une sous-unité IL-12 p40.

6. Le plasmide pour une utilisation dans la méthode de n'importe laquelle des revendications 1 à 5, où la méthode comprend : le fait d'administrer un premier traitement à un premier moment (T1), où le premier traitement comprend le fait d'injecter, dans la tumeur cancéreuse, une première dose efficace dudit plasmide et le fait d'administrer une première thérapie par électroporation à la tumeur au moment T1, la première thérapie par électroporation comprenant l'administration d'au moins une impulsion de tension ayant une durée de 100 microsecondes à 20 millisecondes ; et le fait d'administrer un deuxième traitement à un deuxième moment (T2), où le moment T2 est un moment ultérieur au moment T1, où le deuxième traitement comprend le fait d'injecter, dans la tumeur cancéreuse, une deuxième dose efficace dudit plasmide et le fait d'administrer une deuxième thérapie par électroporation à la tumeur au moment T2, la deuxième thérapie par électroporation comprenant l'administration d'au moins une impulsion de tension ayant une durée de 100 microsecondes à 20 millisecondes.

7. Le plasmide pour une utilisation dans la méthode de la revendication 6, où la méthode comprend en outre : le fait d'administrer un troisième traitement à un troisième moment (T3), où le moment T3 est un moment ultérieur au moment T2, où le troisième traitement comprend le fait d'injecter, dans la tumeur cancéreuse, une troisième dose efficace dudit plasmide et le fait d'administrer une troisième thérapie par électroporation à la tumeur, la troisième thérapie par électroporation comprenant l'administration d'au moins une impulsion de tension ayant une durée de 100 microsecondes à 20 millisecondes.

8. Le plasmide pour une utilisation dans la méthode de la revendication 6, où la méthode comprend en outre : le fait d'injecter une dose efficace dudit plasmide dans la tumeur cancéreuse du sujet ; et le fait d'administrer une électroporation au sujet par voie intra-tumorale à l'aide d'au moins une impulsion de tension basse ayant une largeur d'impulsion de 100 microsecondes à 20 millisecondes.

9. Un plasmide codant pour une forme liée à une membrane d'un agoniste de GITR, CD137, ou CD134, l'agoniste étant une forme liée à une membrane de GITR-L, CD137-L, CD134-L, pour une utilisation dans une méthode de traitement de tumeurs distantes et de métastases chez un sujet ayant un cancer, la méthode comprenant : le fait d'injecter, dans une tumeur cancéreuse, une dose efficace dudit plasmide et un acide nucléique encodant au moins une cytokine immunostimulatrice ; et le fait d'administrer une thérapie par électroporation à la tumeur, la thérapie par électroporation comprenant l'administration d'au moins une impulsion de tension de 200 V/cm à 1 500 V/cm sur une largeur d'impulsion de 100 microsecondes à 20 millisecondes.

10. Le plasmide pour une utilisation dans la méthode de la revendication 9, où le cancer est un mélanome.

11. Le plasmide pour une utilisation dans la méthode de la revendication 9 ou de la revendication 10, où le plasmide comprend en outre l'acide nucléique encodant au moins une cytokine immunostimulatrice.

12. Le plasmide pour une utilisation dans la méthode de n'importe laquelle des revendications 9 à 11, où la cytokine immunostimulatrice est sélectionnée dans le groupe constitué de : IL-12, IL-15, et d'une combinaison de IL-12 et IL-15.

13. Le plasmide pour une utilisation dans la méthode de n'importe laquelle des revendications 9 à 12, où l'acide nucléique encodant au moins une cytokine immunostimulatrice encode une sous-unité IL-12 p35 et une sous-unité IL-12 p40.
